# EUROPEAN PATENT APPLICATION

(11) **EP 1 394 545 A1**
(43) Date of publication of application: **03.03.2004**
(21) Application number: 03018656.3
(22) Date of filing: 21.08.2003
(51) Int. Cl.: G01N 33/487, G01N 27/02, C12Q 1/00, G01N 27/49

(54) **Method of determining a haematocrit corrected glucose concentration in whole blood samples wherein the haematocrit concentration is measured by impedance spectroscopy**

(30) Priority: 27.08.2002 US 406066 P
(71) Applicant: Bayer Healthcare, LLC, Tarrytown, New York 10591 (US)
(72) Inventor: Vreeke, Mark, S., Houston, TX 77070 (US); Genshaw, Marvin, A., Elkhart, IN 46514 (US); Melle, Bryan, S., Elkhart, IN 46514 (US)
(74) Representative: Linhart, Angela

(57) **Abstract**

Method of determining the glucose concentration in a whole blood sample by providing an electrochemical sensor adapted to measure glucose and hematocrit concentrations. The hematocrit concentration of the whole blood sample is measured using the electrochemical sensor via electrochemical impedance spectroscopy. The initial glucose concentration of the whole blood sample is measured using the electrochemical sensor. The unbiased glucose concentration in the whole blood sample is calculated using the initial glucose concentration measurement and the hematocrit concentration.

## Description

### FIELD OF THE INVENTION

The present invention relates generally to methods of determining glucose concentration in whole blood samples and, more specifically, methods of determining glucose concentration in whole blood samples using hematocrit concentration.

### BACKGROUND OF THE INVENTION

Individuals have attempted to determine glucose concentration in whole blood samples for a number of years. The determination of glucose concentration in whole blood samples is important in a variety of applications. For example, determining and monitoring glucose concentration are important for diabetics in reducing risks and improving quality of life. The results of such tests can be used to determine what, if any, insulin or other medication needs are to be administered.

Glucose concentration in whole blood samples can be difficult to determine because of the biasing associated with the whole blood hematocrit concentration. The hematocrit concentration is the concentration of red blood cells in the whole blood sample. Variations in the hematocrit concentration of whole blood samples result in bias to the glucose concentration measurements. The bias to the glucose concentrations can be significant such as, for example, a 1% bias per 1% change in the hematocrit concentration.

Typical sensors used to measure the glucose concentration of a whole blood sample are dependent upon the hematocrit concentration thereof. Attempts have been made to minimize or eliminate the bias in the glucose concentration of a whole blood sample by modifying the chemistry in the glucose sensor. One example of modifying the chemistry is lysing-the blood. Lysing the blood involves breaking up the red blood cells and exposing the contents of the cell. By breaking up the red blood cells, the chemical reaction in the sensor that measures the glucose concentration occurs faster. These attempts have generally reduced the effect of the hematocrit concentration upon the measured glucose concentration, but they generally produce less than ideal results in determining the actual glucose concentrations. Another disadvantage of lysing the blood is the additional time needed to perform the testing process. Additionally, lysing the blood may expose contents of cells that potentially interfere with measuring the glucose concentration.

Other attempts have involved removing the red blood cells from the whole blood sample before measuring the glucose concentration. Such removal methods are more complicated than simply measuring the glucose concentration without removing the red blood cells. Additionally, such techniques are more difficult when using smaller amounts of whole blood such as those used in the disposable self-testing market.

Other attempts to minimize or eliminate the bias in the glucose concentration readings have included separately measuring the hematocrit concentration from the glucose concentration. The hematocrit measurements of the whole blood sample have been performed using a conductivity measurement. These attempts have various disadvantages such as requiring separate electrodes to measure the hematocrit and glucose concentrations. Furthermore, while this approach may be suitable for at least some clinical analyzers, it is cost prohibitive for the disposable self-testing market.

It would be desirable to have a method that obtains a more accurate glucose concentration from a whole blood sample.

### SUMMARY OF THE INVENTION

The glucose concentration in a whole blood sample may be determined according to one method by providing an electrochemical sensor adapted to measure glucose and hematocrit concentrations. The hematocrit concentration of the whole blood sample is measured using the electrochemical sensor via electrochemical impedance spectroscopy. The initial glucose concentration of the whole blood sample is measured using the electrochemical sensor. The unbiased glucose concentration in the whole blood sample is calculated using the initial glucose concentration measurement and the hematocrit concentration.

The glucose concentration in a whole blood sample may be determined according to one method by providing an electrochemical sensor adapted to measure glucose and hematocrit concentrations. The hematocrit concentration of the whole blood sample is measured using the electrochemical sensor via electrochemical impedance spectroscopy using an amperometric monitoring system. The initial glucose concentration of the whole blood sample is measured using the electrochemical sensor. The unbiased glucose concentration in the whole blood sample is calculated using the initial glucose concentration measurement and the hematocrit concentration.

### BRIEF DESCRIPTION OF THE DRAWINGS

FIG. 1 is a plot of phase shift versus frequency of whole blood samples with a single glucose concentration and varying hematocrit concentrations.
FIG. 2 is a plot of impedance versus frequency of whole blood samples with a single glucose concentration and varying hematocrit concentrations.
FIG. 3 is a plot of phase shift versus hematocrit concentration of whole blood samples at a single frequency with varying glucose concentrations.
FIG. 4 is a plot of impedance versus hematocrit concentration of whole blood samples at a single frequency with varying glucose concentrations.

### DETAILED DESCRIPTION OF THE ILLUSTRATED EMBODIMENT

The present invention is directed to determining the glucose concentration in whole blood samples. The whole blood sample comprises plasma and red blood cells. In determining the glucose concentration, the hematocrit concentration (red blood cell concentration) of the whole blood sample is also determined because the glucose concentration varies based upon the hematocrit concentration. By compensating for the hematocrit concentration, a more accurate glucose concentration of the whole blood sample can be calculated.

The glucose concentration of the whole blood sample may be determined by using an electrochemical sensor according to one embodiment of the present invention. It is important that the electrochemical sensor provides reliable and reproducible measurements. An example of an electrochemical sensor is a sensor that may be used in an amperometric monitoring system. Examples of an electrochemical sensor that can be used to measure glucose concentrations are those used in Bayer Corporation's Glucometer DEX® and ELITE® systems.

According to one embodiment, an electrochemical sensor comprises an insulating base plate having provided thereon an electrode system. The electrode system comprises at least an electrode for measurement and a counter electrode. On the electrode system, a reaction layer is included that contains a biosensing or reagent material, such as an enzyme, and an electron acceptor. The enzyme of the reaction layer may be combined with a hydrophilic polymer. A cover may be used to mate with the base plate to form a space including the reaction layer. A whole blood sample is introduced into the space via an introducing port. Gas is discharged from the space by the inflow of the whole blood sample via a discharge port. It is believed that the glucose in the whole blood sample reacts with the enzyme by the action of the glucose oxidase carried on the electrodes to produce hydrogen peroxide. A voltage is applied (*e.g.*, 1V) between the electrodes and the electrode for measurement is polarized in the anode direction. By applying a voltage in the anode direction, an oxidizing current for the produced hydrogen peroxide is obtained. This current level corresponds to the concentration of glucose in the whole blood sample.

More details on such an electrochemical sensor may be found in U.S. Patent Nos. 5,120,420 and 5,320,732 which are both incorporated by reference in their entirety. One or more electrochemical sensor may be purchased from Matsushita Electric Industrial Company. It is contemplated that other electrochemical sensors may be used in the present invention. For example, an electrochemical sensor is disclosed in U.S. Patent No. 5,798,031, which is incorporated by reference in its entirety. A further example of an electrochemical sensor that may be used in an amperometric monitoring system is disclosed in U.S. Patent No 5,429,735. It is contemplated that other sensors may be used in the present invention.

The electrochemical sensors may be located in a blood glucose sensor dispensing instrument that is adapted to have loaded therein a sensor pack that includes a plurality of sensors or testing elements. Each of the sensors is adapted to be ejected from the sensor pack. One example of a sensor pack loaded in a sensor dispensing instrument is disclosed in U.S. Patent No. 5,660,791.

To reduce any bias of the initial glucose concentration resulting from variations in the hematocrit concentration of the whole blood sample, the hematocrit concentration is preferably first measured. It is contemplated, however, that the hematocrit concentration may be measured before measuring the initial glucose concentration of the whole blood sample. The hematocrit concentration needs to be measured because the electrochemical sensors used to measure the initial glucose concentration are dependent upon the hematocrit concentration of the whole blood sample. The greatest sensitivity in accurately measuring the glucose concentration of the whole blood sample occurs at higher glucose levels (*e.g.,* 250 or 400 mg/dL). At such glucose levels, a lower or higher percentage of hematocrit (*e.g.*, 20 vol.% or 70 vol.% hematocrit) causes substantial deviation between the initial glucose concentration measurement and the actual glucose concentration as compared to an average hematocrit concentration (*e.g.*, 40 vol.% hematocrit).

It is believed that the impact of a whole blood sample's hematocrit concentration on the measured glucose concentration is consistent in electrochemical sensors in amperometric monitoring systems. This consistency of the hematocrit concentration on the measured glucose concentration occurs in both disposable amperometric monitoring systems and clinical analyzers as well. Thus, the bias, if any, can be corrected by measuring the hematocrit concentration and subsequently adjusting the initial glucose measurement.

The hematocrit concentration is determined using the same electrochemical sensor that determines the initial glucose concentration of the whole blood sample. According to one embodiment, the hematocrit concentration is determined using electrochemical impedance spectroscopy (EIS) that is integrated in the glucose monitoring system, such as the above described amperometric monitoring system. EIS is essentially a solution impedance measurement covering a single frequency or a plurality of frequencies. The higher the measured impedance of the whole blood sample, the higher the concentration of hematocrit therein.

Electrochemical sensors may be used by applying AC waveforms from generally about 1 to about 10,000 Hz. It is contemplated that the AC waveforms may be higher than 10,000 Hz. The AC waveforms may vary in voltage but are generally from about 1 to about 100 mV and, more specifically, from about 3 to about 30 mV. The AC waveforms are either discrete frequencies or co-added waveforms that are subsequently deconvoluted using a Fourier Transform (FT) routine. The Fourier Transform approach is desirable because it significantly reduces measurement time versus other techniques. It is contemplated that techniques other than the Fourier Transform may be used to assist in ultimately determining the hematocrit concentration.

Alternatively, or in addition to using one or more frequency measurements, a phase shift and/or magnitude of an impedance measurement may also be used to measure the hematocrit concentration. The resulting phase shift and/or magnitude of the impedance are measured at each frequency. The AC waveform is superimposed on a bias voltage of, for example, 150mV, the applied potential at which an electrochemical sensor may be operated. By comparing the phase shift and/or magnitude of an impedance measurement, the hematocrit concentration can be determined. It is desirable to use a frequency or frequencies in which the phase shift and/or magnitude of an impedance measurement can be easily differentiated between hematocrit concentrations.

Examples of frequencies that may be used with measuring the phase shift of an impedance measurement include those from about 800 to 900 Hz. It is contemplated that other frequency or frequencies may be used in measuring phase shift of an impedance measurement. Examples of frequencies that may be used with measuring the magnitude of an impedance measurement include those from about 300 to about 10,000 Hz. It is contemplated that other frequency or frequencies may be used in measuring the magnitude of an impedance measurement.

After the hematocrit concentration is determined, it is used to correct the bias, if any, associated with the initial glucose concentration measurement in the whole blood sample. The relationship or bias, if any, between the hematocrit concentration and the initial glucose concentration measurement may be stored in a calibration table. The calibration table is typically generated using a number of hematocrit concentrations and initial glucose concentration measurements. By correcting for any bias caused by the hematocrit concentration on the initial glucose measurement, a more accurate whole blood glucose measurement is determined.

The method for more accurately determining glucose concentrations by reducing or eliminating any bias caused by hematocrit concentration may be performed in disposable self-testing systems. The disposable self-testing systems are often used by end consumers, especially those who are diabetic. Alternatively, the method for more accurately determining glucose concentrations by reducing or eliminating any bias caused by the hematocrit concentration may be performed in clinical analyzers. Clinical analyzers are often used in hospitals or clinics.

The present invention is especially desirable with neonates (newborn children that are less than one month old). Neonates typically have fairly high blood hematocrit concentrations (normal ranges from about 55 vol.% to 65 vol.%). One of medical conditions that may occur in neonates, as well as adults, is hypoglycemia. With respect to hypoglycemia, the decision point in neonates is lower than that of adults (40 mg/dL vs. 60 mg/dL). The combination of lower glucose concentrations with higher hematocrit concentrations may lead to highly inaccurate initial glucose readings caused from the bias of the hematocrit concentrations. Thus, the present invention is desirable in determining the glucose concentration in the whole blood sample of neonates.

The testing end of the sensor is adapted to be placed into contact with the whole blood sample to be tested. The whole blood sample may be generated by a lancing device such as a microlet. The lancing device may obtain blood by, *e.g.*, pricking a person's finger. According to one process, the whole blood sample may be prepared for testing by (a) removing the electrochemical sensor from packet, (b) placing the electrochemical sensor into a glucose concentration measuring instrument, (c) generating a whole blood sample and (d) bringing the sensor and the whole blood sample into contact wherein the blood is generally drawn into the sensor by capillary action.

### Examples

Several plots have been prepared to show the relationships between (a) hematocrit level and (b) phase shift, glucose concentration, frequency and impedance.

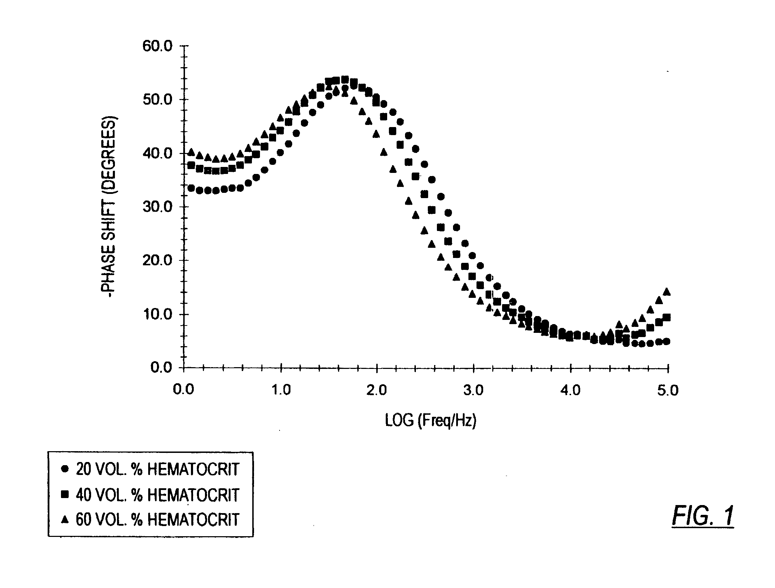

FIG. 1 is a plot of phase shift versus frequency of whole blood samples. The whole blood samples had a glucose concentration of 300 mg/dL and the hematocrit concentration was either 20 vol.%, 40 vol.% or 60 vol.%. The tests were performed on Bayer Corporation's Glucometer DEX® system with a three-pass reagent electrochemical sensor. The electrochemical sensor was operated at an applied potential of 150mV. The frequency (measured in Hertz) was plotted logarithmically along the x-axis versus the phase shift (measured in degrees) which was plotted along the y-axis. FIG. 1 shows that there are many frequencies that result in distinct phase shifts between varying hematocrit concentrations. It is desirable to chose a frequency in which the phase shift is more pronounced between varying levels of hematocrit concentrations.

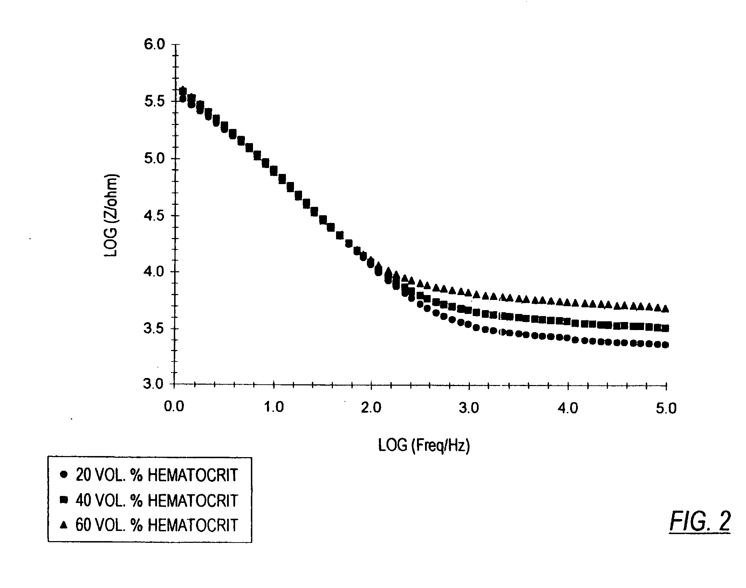

FIG. 2 is a plot of impedance (Z) versus frequency of whole blood samples. The whole blood samples had a glucose concentration of 100 mg/dL and the hematocrit concentration was either 20 vol.%, 40 vol.% or 60 vol.%. The tests were performed on Bayer Corporation's Glucometer DEX® system with a three-pass reagent electrochemical sensor. The electrochemical sensor was operated at an applied potential of 150mV. The frequency (measured in Hertz) was plotted logarithmically along the x-axis versus the impedance (measured in ohms) which was plotted logarithmically along the y-axis. FIG. 2 shows that there are many frequencies that result in distinct impedance measurements between varying hematocrit concentrations. It is desirable to chose a frequency, such as the higher frequencies, in which the impedance measurements are more pronounced between varying levels of hematocrit concentrations.

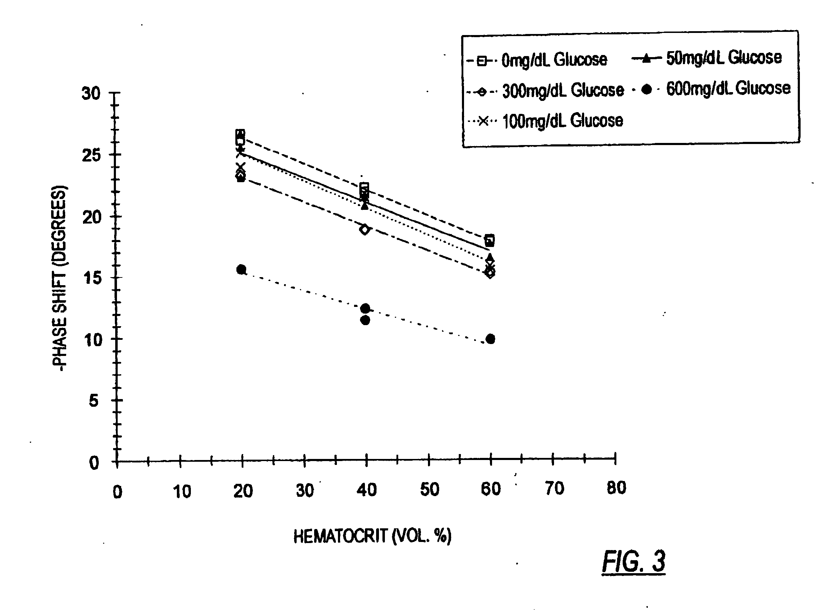

FIG. 3 is a plot of phase shift versus hematocrit concentration of whole blood samples. There were several glucose concentrations that were tested: 0 mg/dL, 50mg/dL, 100 mg/dL, 300 mg/dL and 600 mg/dL. Each of the glucose concentrations was plotted separately in FIG. 3. The individual data points that were used in forming the plot of FIG. 3 are listed in Table 1 below.

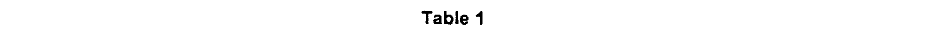

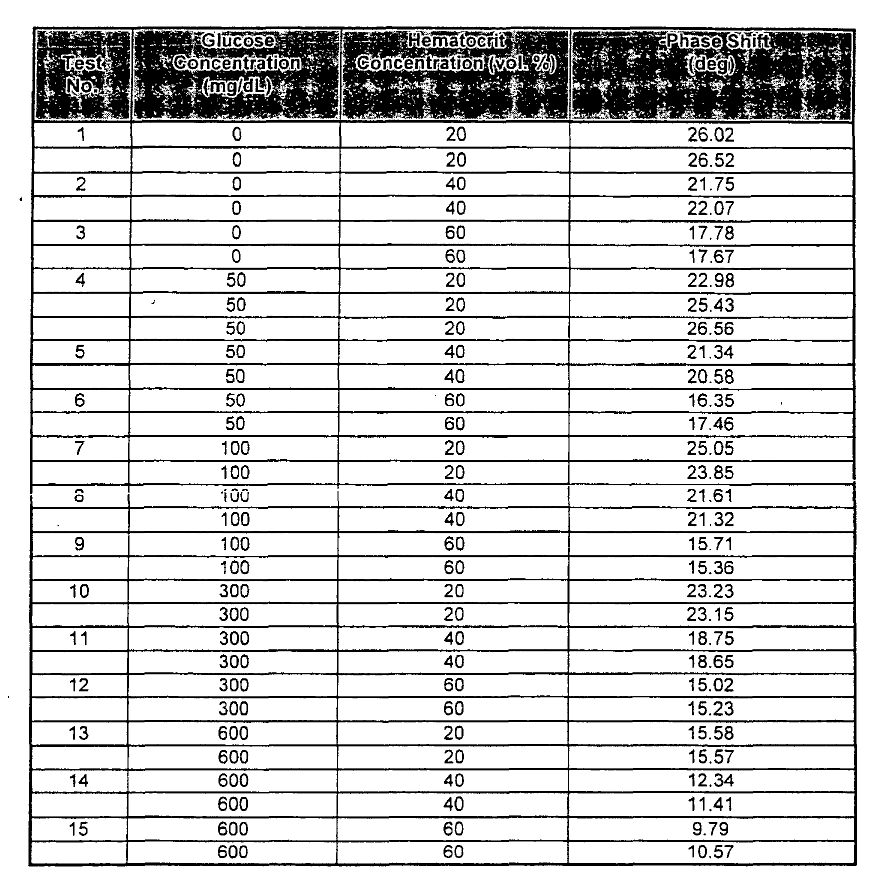

As shown in Table 1, almost all of the recited glucose concentration and hematocrit concentration combinations were tested twice. The testing of the phase shift versus hematocrit concentrations reported in Table 1 and plotted in FIG. 3 was done at a frequency of 811 Hz. The tests were performed on Bayer Corporation's Glucometer DEX® system with a three-pass reagent electrochemical sensor. The electrochemical sensor was operated at an applied potential of 150mV. The hematocrit concentration (vol.%) was plotted along the x-axis versus the phase shift (measured in degrees) which was plotted along the y-axis. FIG. 3 shows that there is a variation in the phase shift with glucose concentration at a frequency of at least 811 Hz. FIG. 3 also shows that there is a variation in the phase shift with respect to the hematocrit concentration.

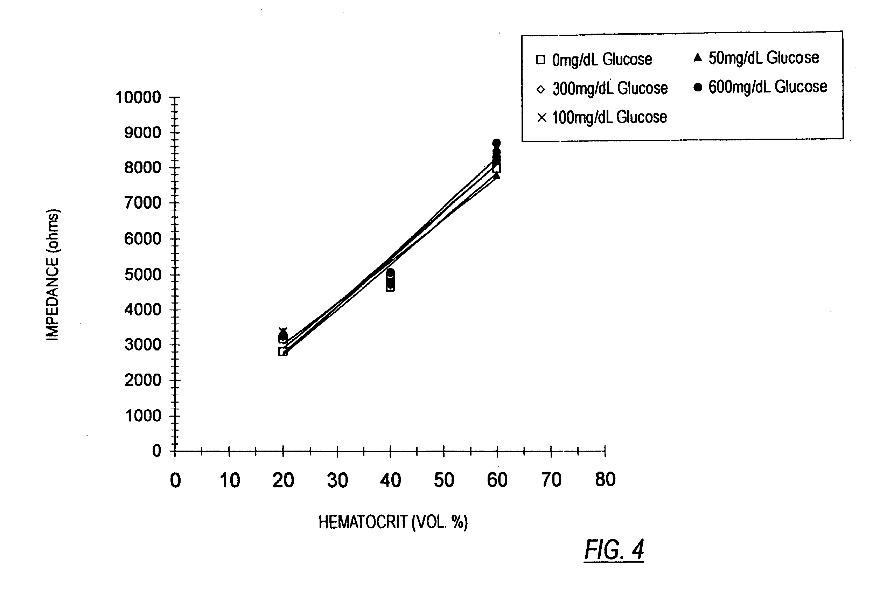

FIG. 4 is a plot of impedance (Z) versus hematocrit concentration of whole blood samples. There were several glucose concentrations that were tested: 0 mg/dL, 50mg/dL, 100 mgl/dL, 300 mg/dL and 600 mg/dL. Each of the glucose concentrations was plotted separately in FIG. 4. The individual data points that were used in forming the plot of FIG. 4 are listed in Table 2 below.

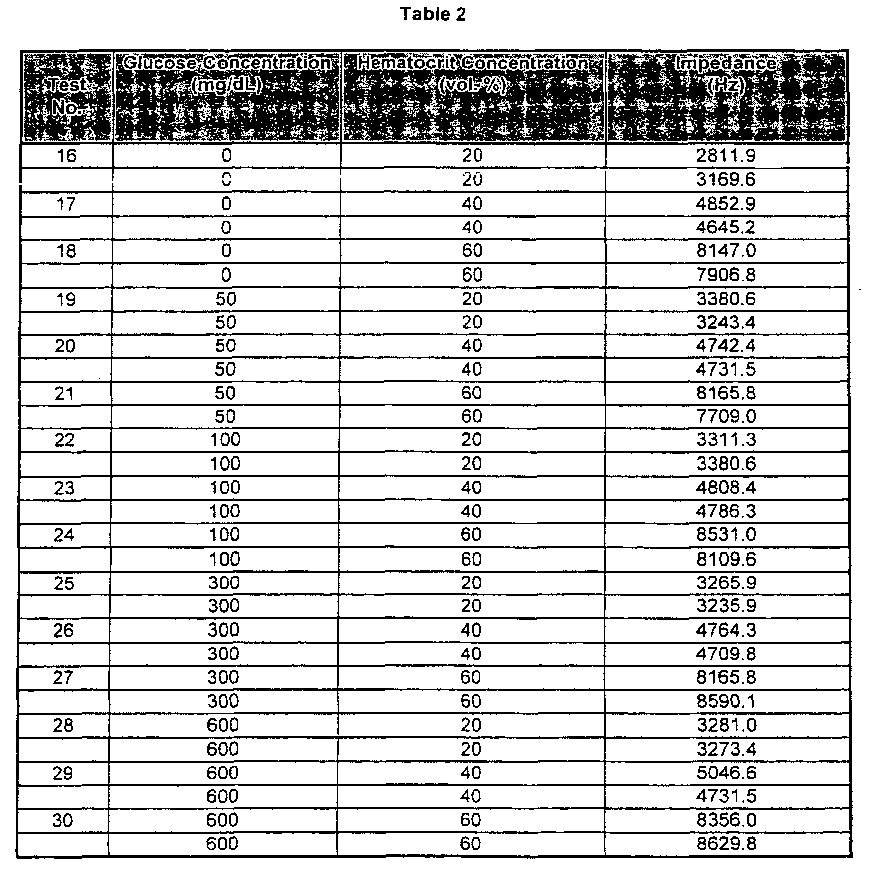

As shown in Table 2, all of the recited glucose concentration and hematocrit concentration combinations were tested twice. The testing of the impedance versus hematocrit concentrations reported in Table 2 and plotted in FIG. 4 was done at a frequency of 9661 Hz. The tests were performed on Bayer Corporation's Glucometer DEX® system with a three-pass reagent electrochemical sensor. The electrochemical sensor was operated at an applied potential of 150mV. The hematocrit concentration (vol.%) was plotted along the x-axis versus the impedance (measured in ohms) which was plotted along the y-axis. FIG. 4 shows that there is a variation in impedance with respect to the hematocrit concentration at a frequency of at least 9661 Hz. FIG. 4 appears to show that the magnitude of the impedance is independent of the glucose concentration at a frequency of at least 9661 Hz.

While particular embodiments and applications of the present invention have been illustrated and described, it is to be understood that the invention is not limited to the precise construction and compositions disclosed herein and that various modifications, changes, and variations may be apparent from the foregoing descriptions without departing from the spirit and scope of the invention as defined in the appended claims.

## Claims

1. A method of determining glucose concentration in a whole blood sample comprising:
providing an electrochemical sensor adapted to measure glucose and hematocrit concentrations;
measuring the hematocrit concentration of the whole blood sample using the electrochemical sensor via electrochemical impedance spectroscopy;
measuring the initial glucose concentration of the whole blood sample using the electrochemical sensor; and
calculating the unbiased glucose concentration in the whole blood sample using the initial glucose concentration measurement and the hematocrit concentration.

2. The method of claim 1, wherein the glucose concentration of the whole blood sample is determined using an amperometric monitoring system.

3. The method of claim 1, wherein the electrochemical sensor includes an insulating base plate, an electrode system on the base plate and a cover adapted to mate with the base plate to form a space in which the electrode layer is available to contact the whole blood sample.

4. The method of claim 3 further including a reaction layer comprising an enzyme that reacts with the glucose in the whole blood sample.

5. The method of claim 4, wherein the enzyme in the reaction layer is combined with a hydrophilic polymer.

6. The method of claim 1, wherein the method of determining glucose concentration in a whole blood sample occurs in disposable self-testing systems.

7. The method of claim 1, wherein the method of determining glucose concentration in a whole blood sample occurs in a clinical analyzer.

8. The method of claim 1, wherein the measuring of the hematocrit concentration in the whole blood sample is performed before measuring the initial glucose concentration.

9. The method of claim 1, wherein the measuring of the hematocrit concentration of the whole blood sample is performed using a single frequency measurement.

10. The method of claim 1, wherein the measuring of the hematocrit concentration of the whole blood sample is performed using a plurality of frequency measurements.

11. The method of claim 1, wherein the measuring of the hematocrit concentration is performed using a phase shift of an impedance measurement.

12. The method of claim 11, wherein the measuring of the hematocrit concentration is performed with at least one frequency between about 800 and about 900 Hz.

13. The method of claim 1, wherein the measuring of the hematocrit concentration is performed using magnitude components of an impedance measurement.

14. The method of claim 13, wherein the measuring of the hematocrit is performed with at least one frequency between about 300 and about 10,000 Hz.

15. The method of claim 1 further including applying AC waveforms from about 1 to about 10,000 Hz to the electrochemical sensor.

16. The method of claim 1 further including applying AC waveforms from about 1 to about 100mV to the electrochemical sensor.

17. The method of claim 1 further applying AC waveforms that are subsequently deconvoluted using a Fourier transform.

18. A method of determining glucose concentration in a whole blood sample comprising:
providing an electrochemical sensor adapted to measure glucose and hematocrit concentrations;
measuring the hematocrit concentration of the whole blood sample using the electrochemical sensor via electrochemical impedance spectroscopy using an amperometric monitoring system;
measuring the initial glucose concentration of the whole blood sample using the electrochemical sensor; and
calculating the unbiased glucose concentration in the whole blood sample using the initial glucose concentration measurement and the hematocrit concentration.

19. The method of claim 18, wherein the method of determining glucose concentration in a whole blood sample occurs in disposable self-testing systems.

20. The method of claim 19, wherein the measuring of the hematocrit concentration of the whole blood sample is performed using a single frequency measurement.

21. The method of claim 19, wherein the measuring of the hematocrit concentration of the whole blood sample is performed using a plurality of frequency measurements.

22. The method of claim 19, wherein the measuring of the hematocrit concentration is performed using a phase shift of an impedance measurement.

23. The method of claim 22, wherein the measuring of the hematocrit concentration is performed with at least one frequency between about 800 and about 900 Hz.

24. The method of claim 19, wherein the measuring of the hematocrit concentration is performed using magnitude components of an impedance measurement.

25. The method of claim 24, wherein the measuring of the hematocrit is performed with at least one frequency between about 300 and about 10,000 Hz.

26. The method of claim 19 further including applying AC waveforms from about 1 to about 10,000 Hz to the electrochemical sensor.

27. The method of claim 19 further including applying AC waveforms from about 1 to about 100mV to the electrochemical sensor.

28. The method of claim 19 further applying AC waveforms that are subsequently deconvoluted using a Fourier transform.
